# EUROPEAN PATENT APPLICATION

(11) **EP 4 360 678 A1**
(43) Date of publication of application: **01.05.2024**
(21) Application number: 22306633.3
(22) Date of filing: 28.10.2022
(51) Int. Cl.: A61M 5/32

(54) **A SAFETY DEVICE FOR MOUNTING ONTO A DRUG DELIVERY DEVICE**

(71) Applicant: Becton, Dickinson and Company, Franklin Lakes, NJ 07417-1880 (US)
(72) Inventor: ALBENGE, Olivier, 38130 Echirolles (FR); CHANSAVANG, Albert, 38000 Grenoble (FR); MILLS, Freddy, 38000 Grenoble (FR); BESSON, Nicolas, 38650 Treffort (FR)
(74) Representative: Germain Maureau

(57) **Abstract**

The safety device (10) includes a tubular body (12) extending along a longitudinal axis (A), the tubular body being configured to receive the barrel, an annular head (14) configured to abut against the flange; one actuation arm connecting the tubular body and the annular head, the actuation arm being movable by a user between a collapsed position in which the tubular body is maintained by the actuation arm in a position proximally close to the flange uncovering the needle and an extended position in which the tubular body is maintained in a position distally distant from the flange shielding the needle; one finger resting arrangement connected to the annular head; a locking means for maintaining the actuation arm in a collapsed position and a locking means for maintaining the actuation arm in an extended position.

## Description

The present invention relates to a safety device for mounting onto a drug delivery device such as a prefilled or pre-fillable syringe in order to protect a user from needle stick injuries after injection of a medical product. The invention also relates to a drug delivery device including this safety device.

### Background

In this application, the distal end of a component or of a device is to be understood as meaning the end furthest from the user's hand and the proximal end is to be understood as meaning the end closest to the user's hand. Likewise, in this application, the "distal direction" is to be understood as meaning the direction of injection, with respect to the safety device or drug delivery device of the invention, and the "proximal direction" is to be understood as meaning the opposite direction to said direction of injection, that is to say the direction towards the user's hand.

Drug delivery devices, such as pre-fillable or prefilled syringes, usually comprise a hollow body or barrel forming a container for a medical product. This body comprises a distal end in the form of a longitudinal tip defining an axial passageway through which the medical product is expelled from the container. The distal end is equipped with a needle for injection of the medical product into an injection site.

It is of great importance that the patients and users are protected from any risk of needle stick injuries, particularly between the moment that injection is finished and the discarding of the drug delivery device.

In order to minimize the risks of needle stick injuries, drug delivery devices may be equipped with a safety device that protects the needle after injection. Safety devices usually comprise a tubular body for receiving the syringe barrel, and a needle cover, in the form of a protective sleeve, that slides relative to the tubular body. The needle cover has a retracted position in which the needle cover is substantially contained inside the tubular body to allow a user to carry out an injection, and an extended position in which the needle cover moves distally from the retracted position to cover the needle once the injection is completed.

Although known injection systems are generally satisfactory, they do not always meet all of the user's expectations and they can include a number of components. Therefore, they can be expensive to manufacture and thus this can limit the widespread use of safety devises at the expense of the security of medical users and patients.

They also tend to include components made of various materials which can be complex and expensive to recycle.

### Summary

A need exists for an improved safety that provides a user with more convenient safety device that is also cost effective and easy to manufacture.

In one aspect, the present invention is directed to a safety device for mounting onto a drug delivery device having a barrel including with a flange at its proximal end and an injection needle at its distal end, and a piston rod including a piston flange. The safety device includes:
a tubular body extending along a longitudinal axis (A), the tubular body being configured to receive the barrel,
an annular head configured to abut against the flange,
one actuation arm connecting the tubular body and the annular head, said actuation arm having a proximal link connected to the annular head by a proximal hinge and a distal link connected to the tubular body by a distal hinge, the proximal link and the distal link being connected by an intermediate hinge, the actuation arm being movable between a collapsed position in which the tubular body is maintained in a position proximally close to the flange uncovering the needle and an extended position in which the tubular body is maintained in a position distally distant from the annular head shielding the needle;
one finger resting arrangement connected to the annular head;
a locking means for maintaining the actuation arm in a collapsed position and a locking means for maintaining the actuation arm in an extended position.

The proximal link can include at least one locking hooks configured and sized to enter into resilient snap fit engagement with at least one wings protruding from the annular head to maintain the actuation arm in the extended position.

The annular head can comprise a locking member which includes a radially oriented wall and two wings extending laterally from the wall.

The annular head can include a longitudinal tongue which extends distally from the locking member and is configured and sized to engage into a channel defined by two guiding walls provided on the tubular body.

The longitudinal tongue can include at least one teeth configured and sized to engage into on opening provide on the tubular body.

The annular head can include a tab which extends distally from the locking member, the tab including a transverse corrugation configured to engage and sized on a rib provided on the annular body.

The intermediate hinge can inwardly overtake the plan defined by the proximal hinge and distal hinge when the actuation arm is in its extended position.

The annular head can include a proximal ring and a distal ring jointed by a shoulder whereon the flange abuts.

At least one the hinge can be a living hinge.

The annular head can comprise at least one locking tab configured to retain the flange.

The tubular body can comprises a leg extending in cantilever and having a catch at its free end, the leg being configured to deflect radially outward to allow the catch to slide over the annular head during mounting and to deflect radially inward after mounting completion so that the catch axially retains the tubular body relative to annular head.

The finger receiving member can comprise a longitudinal finger receiving plate and a radial finger receiving plate.

The longitudinal finger receiving plate can comprise at least one corrugation configured to engage onto a rib protruding from the tubular body when the actuation arm is in its collapsed state.

The longitudinal finger receiving plate can comprise at least one guiding tab extending from its inner face and configured to slidably engage with at least one guiding groove provided on the tubular body.

At least one guiding tab can include a teeth positioned at its free end, the teeth being configured and sized to snap in a recess provided in the guiding groove.

The annular head can comprise a tongue which extends distally in cantilever, configured to engage in a channel defined between two parallel guiding wall extending outwardly from the tubular body.

The tongue can include at least one teeth configured to engage into a corresponding window made in the tubular body.

The distal link can comprise an arm extending from the distal link inner face, having at its free end a tab configured to enter into resilient snap fit engagement with the locking hooks.

The safety device can comprise a T-shaped rib formed along the inner face of the finger resting arrangement configured and sized to slide in a complementary T-shaped groove longitudinally formed along the tubular body.

The safety device can be formed in a single shot injection molding process.

The safety device may include at least one recess positioned at the junction between the shoulder and the distal ring.

In a second aspect, the invention is directed to a drug delivery device having a barrel including with a flange at its proximal end and an injection needle at its distal end, and a piston rod including a piston flange fitted with a safety device as previously described, wherein the piston flange includes a ramped surface distally convergent configured to outwardly deflect the locking means maintaining the actuation arms in a collapsed position.

### Brief description of the drawings

With reference to the appended drawings, below follows a more detailed description of aspects and embodiments of the invention cited as examples.
Fig.1 is a perspective view of a safety device according to one embodiment of the invention mounted on a drug delivery device;
Fig.2 is an exploded view of the safety device of Fig.1 ;
Fig.3 is a perspective view of the safety device of Fig.1;
Fig.4 is a perspective view of the safety device with an actuation arm thereof removed for clarity;
Fig.5 is a perspective view of the safety device of Fig.1 where the actuation arm is in an extended state;
Fig.6 is partial perspective view of the safety device of Fig.1;
Fig.7 and Fig.8 are views of exemplary use a safety device according to the invention;
Fig.9 is a perspective view of another embodiment of a safety device according to the invention;
Fig.10 and Fig.10a are perspective views of another embodiment of a safety device according to the invention;
Fig. 11 and Fig. 11a are perspective views of another embodiment of a safety device according to the invention;
Fig.12 and Fig.12a are perspective views of another embodiment of a safety device according to the invention;
Fig.13 and Fig.13a are perspective views of another embodiment of a safety device according to the invention;
Fig.14 and Fig.14a are perspective views of another embodiment of a safety device according to the invention;
Fig.15 and Fig.15a are perspective views of another embodiment of a safety device according to the invention;
Fig.16 and Fig.16a are perspective views of another embodiment of a safety device according to the invention;
Fig.17 and Fig.17a are perspective views of another embodiment of a safety device according to the invention;
Figs. 19 to 23 are perspective views of another embodiment of a safety device according to the invention.

### Description of the invention

The different features of the embodiments can be used in combination with and used with other embodiments as long as the combined parts are not inconsistent with or interfere with the operation of the device and assembly. This invention is not limited in its application to the details of construction and the arrangement of components set forth in the following description or illustrated in the drawings. The embodiments herein are capable of being modified, practiced or carried out in various ways. The phraseology and terminology used herein is for the purpose of description and should not be regarded as limiting. The use of "including," "comprising," or "having" and variations thereof herein is to encompass the items listed thereafter and equivalents thereof as well as additional items. Unless limited otherwise, the terms "connected," "coupled," and "mounted," and variations thereof herein are used broadly and encompass direct and indirect connections, couplings, and mountings. In addition, the terms "connected" and "coupled" and variations thereof are not limited to physical or mechanical connections or couplings. Further, terms such as distal, proximal, up, down, bottom, and top are relative, and are to aid illustration, but are not limiting. The embodiments are not intended to be mutually exclusive so that the features of one embodiment can be combined with other embodiments as long as they do not contradict each other. Terms of degree, such as "substantially", "about" and "approximately" are understood by those skilled in the art to refer to reasonable ranges around and including the given value and ranges outside the given value, for example, general tolerances associated with manufacturing, assembly, and use of the embodiments. The term "substantially" when referring to a structure or characteristic includes the characteristic that is mostly or entirely present in the structure.

With reference to the Figures, a safety device 10 is configured to be mounted onto a drug delivery device 100 to protect an injection needle 101 of the drug delivery device 100 after an injection has been carried out.

The drug delivery device 100 may be a prefilled or prefillable syringe. The drug delivery device 100 includes a tubular barrel 102 defining a reservoir for containing a medical product such as a drug. The tubular barrel 102 may be made of a plastic or a glass material. This barrel 102 has a distal shoulder 103 provided with a distal tip 104 longitudinally protruding from said shoulder 103 along a longitudinal axis A. The distal tip 104 defines an axial passageway in fluid communication with the reservoir and is equipped with the injection needle 101 for injecting the medical product in an injection site. The barrel 102 further includes an opposite opened proximal end 106 provided with a flange 107. The opened proximal end 106 receives a plunger rod 110 for pushing a stopper 112 located inside the barrel 102 to expel the medical product from the reservoir to the injection site via the distal tip 104 and the injection needle 101.

The drug delivery device 100 may be fitted with a tip cap 113 for protecting and sealing the injection needle 101 before injection. The tip cap 113 may be comprised a soft inner element 115 and a rigid outer element 116.

The piston rod 110 is fitted with a plunger flange 114 at its proximal end. In some embodiments, the plunger flange 114 can include a ramped surface 118. The ramped surface 118 is conically convergent toward the distal direction.

The safety device 10 includes a tubular body 12 which extends along the longitudinal axis A and is configured and sized to engage on the barrel 102 and an annular head 14 configured and size to engage on the barrel 102 and to lock on the flange 107.

The tubular body 12 and the annular head 14 are connected by one articulated actuation arm 16.

The safety device 10 can be a single piece component. Further, the safety device 10 can be formed by a single shot molding process. The plastic polymer used in the molding process can be a suitable polymer such as thermoplastic.

The tubular body 12 is a tubular element, which has an inner diameter sized and configured so that the tubular body 12 can axially slide relative to the barrel 102. In practice, the tubular body 12 inner diameter slightly exceeds the external diameter of the barrel 102. In some embodiments, the tubular body 12 can be provided with one or more windows 19 so that any marking or labelling applied on the barrel 102 and so that the drug contained in the barrel 102 can be visible to a user prior to injection. In the illustrated embodiments, the tubular body 12 is provided with two windows 19 spaced apart by 180°.

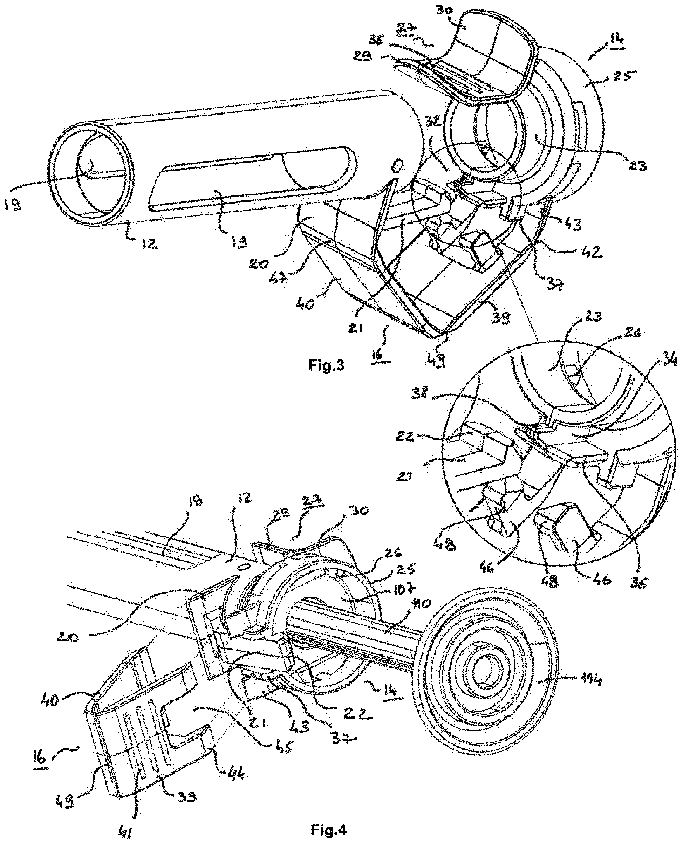

In some embodiments, the tubular body 12 can comprise a tab 20, which is positioned at its proximal end. The tab 20 extends radially from the outer surface of the tubular body 12 and as it can be seen on Fig. 3, the tab 20 can be oriented substantially perpendicular to the longitudinal axis A.

The tubular body 12 further comprises a leg 21. The leg 21 extends in cantilever from the tab 20 in the proximal direction. In some embodiments, the leg 21 can have an arched shape with an inward facing concavity. The cantilevered attachment of the leg 21 onto the tab 20 combined with its arched shape provide the leg 21 with elastic resilience relative to the tubular body 12 in a radial direction. At the end of the leg 21, is a catch 22, which is internally oriented. In some embodiments, the catch 22 has a ramped end.

Now turning to Figs. 3 to 5, the annular head 14 comprises a distal ring 23 attached by a shoulder 24 to a proximal ring 25. The distal ring 23 is coaxial with the tubular body 12 and has an inner diameter, which substantially equates the inner diameter of the tubular body 12.

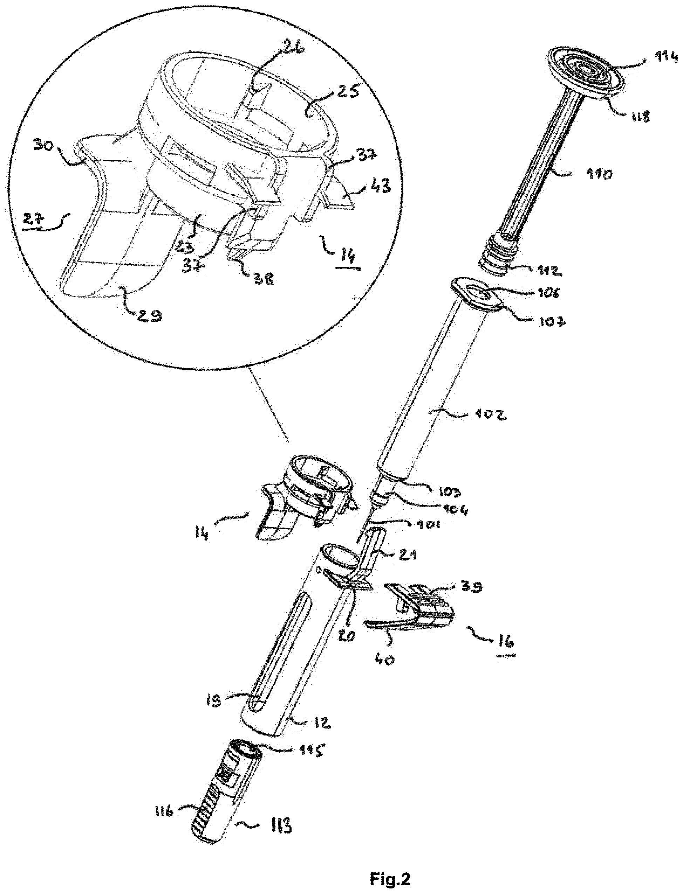

As can be seen on Fig.2, the distal ring 23 can be provided with one or more locking tabs 26, which radially protrude from the inner surface of said distal ring 23. In the illustrated embodiments, the distal ring 23 includes four locking tabs 26 spaced apart by 90°.

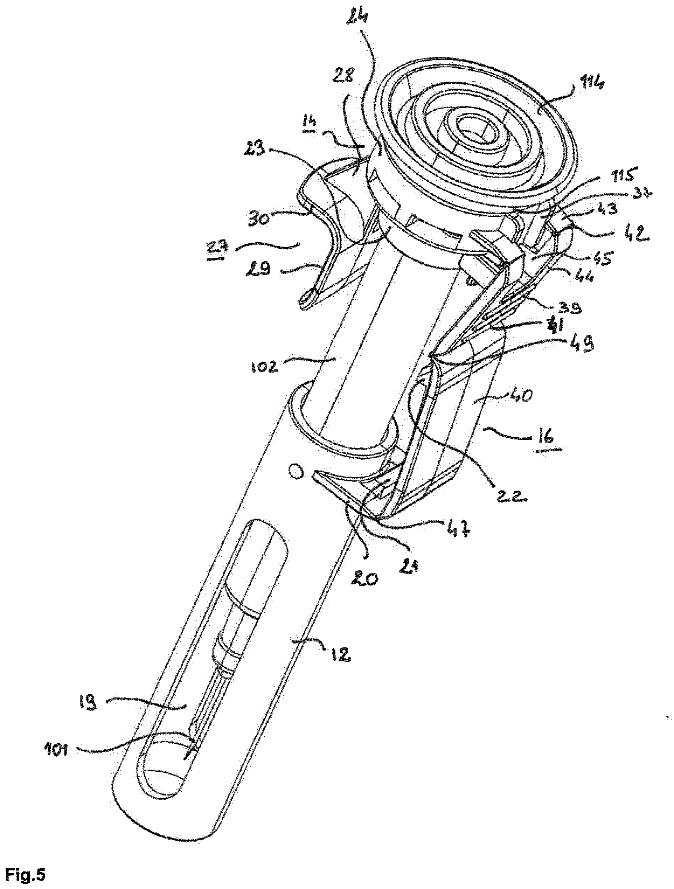

The annular head 14 further comprises a finger receiving member 27 which is fixed to the distal ring 23 preferably by injection molding. The finger receiving member 27 can extend in cantilever from the distal ring 23 and the shoulder 24. Fig 5 shows a wall 28 connecting the finger receiving member 27 to the shoulder 24 and distal ring 23. The finger receiving member 27 can include a longitudinal finger receiving plate 29 which is substantially longitudinally oriented and parallel to the longitudinal axis A and a radial finger receiving plate 30 which is substantially perpendicular to the longitudinal finger receiving plate 29. The first receiving plate 29 can be provided with a series of ribs 35. It can be noted that the free end of the longitudinal finger receiving plate 29 can have an arched shape.

In some embodiments, the annular head 14 further comprises a projecting locking member 32 diametrically opposite the finger receiving member 27 which is best seen in the zoomed part of Fig.3. The projecting locking member 32 which extends from the shoulder 24 and the distal ring 23 has substantially T shaped cross section and includes a radially oriented wall 34 which protrudes from the distal ring 23. At its free end the wall 34 is provided with two wings 36 which extend from the wall 34 in a substantially perpendicular direction. The projecting locking member 32 further includes a ramped nose 38 which tapers in a distal direction. In some embodiments, the projecting locking member 32 includes two parallel guiding ears 37. The guiding ears 37 protrude from the distal ring 23 on the side of the wings 36.

The annular head 14 and the tubular body 12 are linked by the articulated actuation arm 16 which includes a proximal link 39 and a distal link 40.

The proximal link 39 has a substantially rectangular shape and is connected to the annular head 14 by a first proximal hinge 42. The annular head 14 has two spaced apart arms 43 which are connected to two corresponding arms 44 made in the proximal link 39. The first proximal hinge 42 thus connects the end of the two arms 43 and the two arms 44 of the proximal link 39. The two arms 44 define a window 45 in the proximal link 39. In the illustrated embodiment, the first proximal hinge 42 is a living hinge formed by a thinning of the plastic material which the safety device 10 is made of. This allows the proximal link 39 to rotate relative to the arms 43 and 44. In some embodiments, the proximal link 39 is provided with a series of grooves 41. The proximal link 39 further comprises two locking hooks 46 which protrude from its internal face. The two locking hooks 46 are sized and shaped to create a snap fit engagement with the wings 36 of the projecting locking member 32. To this end, the locking hooks 46 can include a ramped finger 48 and the wings 36 can also have a tapered cross section.

The distal link 40 has a substantially rectangular shape and is connected to the tab 20 by a second distal hinge 47 which can be a living hinge made by a thinning of the plastic material which forms the safety device 10. In some embodiments, the distal link 40 can have a length higher than the proximal link 39 length.

The proximal link 39 and the distal link 40 are joined by an intermediate hinge 49 which can be a living hinge made by a thinning of the plastic material which forms the safety device 10.

Thus in some embodiments, the safety device 10 comprises an articulated actuation arm 16 which connects the tubular body 12 and the annular ring 14 and comprises a unitary structure integrally molded of thermoplastic material.

In some embodiments, in an initial state, (see e.g. Fig 3), after manufacturing by injection molding, the safety device 10 is configured in a state wherein the annular head 14 and the tubular body 12 are axially spaced apart while the proximal link 39 and the distal link 40 form a substantially right angle.

In an exemplary use, the safety device 10 can be engaged on a drug delivery device 100 which can be a prefilled syringe as illustrated on the Figs.

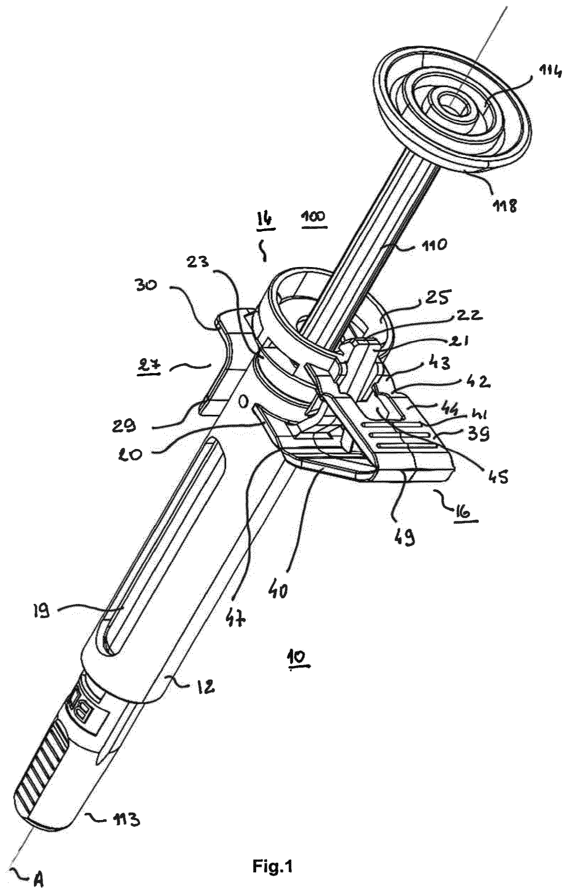

The drug delivery device distal end is inserted through the annular head 14 and is pushed in a distal direction (see e.g. Fig. 1). The annular head 14 is locked onto the barrel flange 107. As best seen on Fig 4, the locking tabs 26 deflect when the annular head 14 is pushed in a proximal direction so that the annular head 14 is retained on the barrel flange 107.

The drug delivery device 100 is axially locked on the safety device 10, however the drug delivery device 100 is free to rotate relative to the safety device 10, which can be useful as users tend to rotate any drug delivery device before injection to visually check the drug which is about to be injected.

Further, the tubular body 12 is pushed in a proximal direction so that the leg 21 which extends from said tubular body 12 deflects against the nose 38, this being made smooth by the ramped catch 22 deflecting against the tapered nose 38. The leg 21 is then channeled between the guiding ears 37. At the end of the push, the catch 22 locks against the free end of the proximal ring 25 achieving an appropriate retention of the tubular body 12 onto the annular ring 14. In this locked state, the actuation arm 16 is pushed in a collapsed state. In some embodiments, the actuation arm 16 in its collapsed state has the proximal link 39 substantially perpendicular to the axis A.

The drug delivery device fitted with a safety device 100 as illustrated on Fig. 1 can be stored for a later use.

When an injection of the drug contained in the drug delivery device 100 is required, a user may first remove the tip cap 113.

The drug delivery device 100 is ready for injection. The user can visually check the drug through the windows 19 and can rotate the plunder rod 110.

Administering an injection with a drug delivery device fitted with the safety device 10 is substantially the same as administering an injection with a drug delivery device fitted with the safety device 10 according to the prior art and thus is not likely to confuse a user and permits a user-friendly operation.

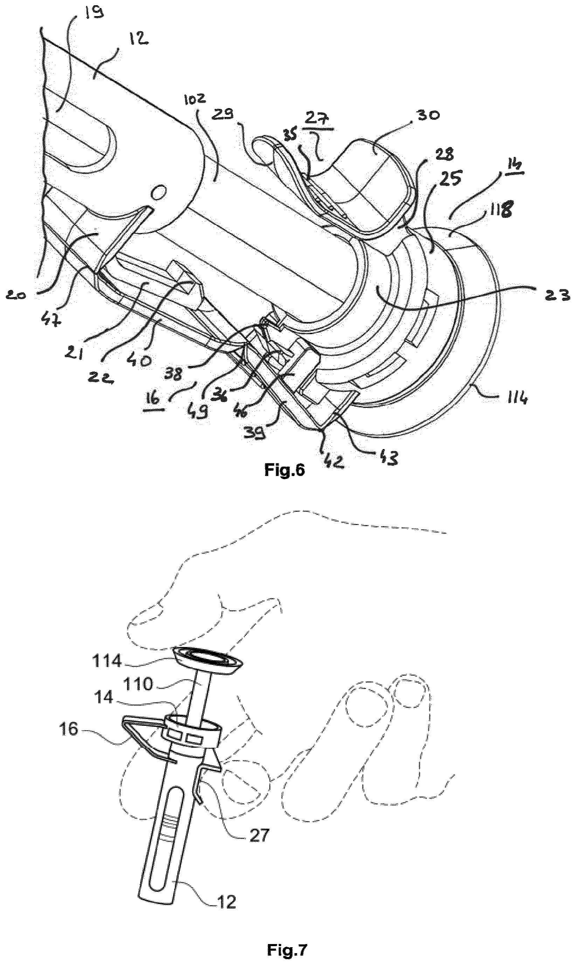

In an exemplary use illustrated on Fig.7, the user can apply a pushing force on the piston rod flange 114 typically by applying a thumb on the piston rod flange 114 while the user maintains one finger on the finger receiving member 27 and one finger on the actuation arm 16, more specifically, the user can keep a finger in the area of the second hinge 47.

As the injection progresses, the piston rod flange 114 moves closer to the annular head 14. At the end of the injection, the piston rod flange 114 abuts against the catch 22. By further pushing the piston rod flange 114, the ramp portion 118 comes in abutment with the ramp provided in the catch 22. The leg 21 is thus forced to deflect outwardly thereby releasing the catch 22. The actuation arm 16 and the tubular body 12 are released from the annular head 14 and are therefore free to move axially along the barrel 102.

In one or more embodiment, the actuation arm 16 may move toward its initial state where the proximal link 39 and the distal link 40 form a substantially right angle depending on the material and the injection process used to manufacture the safety device 10.

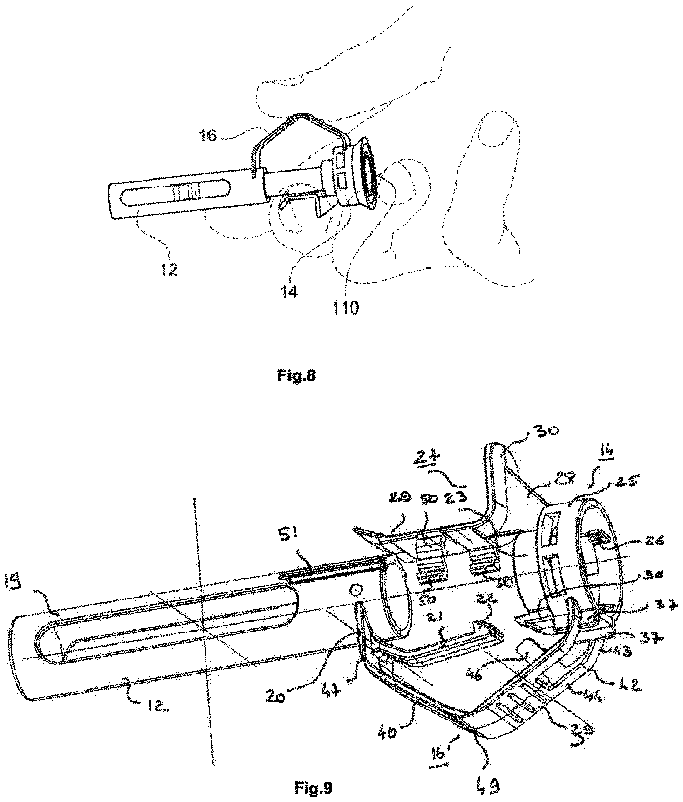

In one exemplary use illustrated on Fig.8, the user can then put a finger over the finger receiving element 27 and pushes on the actuation arm 16 with a second finger. The user can push on the intermediate hinge 49 which is an intuitive pushing point as it protrudes radially so that both proximal link 39 and distal link 40 rotationally move towards the barrel 102.

By doing so, the actuation arm 16 moves into an extended state moving distally the tubular body 12 into a position where the tubular body 12 encapsulates the needle 101.

At the end of the pushing on the actuation arm 16, the two locking hooks 46 lock into the wings 36 of the projecting locking member 32 and maintain the actuation arm 16 in its extended position where the tubular body 12 is pushed distally and thus the tubular body 12 shields the needle 101 from post-injection needle stick.

In some embodiments, the engagement of the locking hooks 46 on the wings 36 produces an audible and/or tactile signal indicating that locking is positively achieved.

In some embodiments, the actuation arm 16 is in a state where the intermediate hinge 49 inwardly overtakes the plan defined by the proximal hinge 42 and distal hinge 47 as it can be best seen on Figs. 5 and 6. Thus, a force applied longitudinally on the tubular body 12 aiming at un-shielding the needle tip 101 cannot release the actuation arm 16 from its extended state. The tubular body 12 is locked in an extended state by the locking hooks 46; locking is further achieved by the proximal link 39 and distal link 40 which are arranged as a toggle mechanism.

A substantially permanent locking of the tubular body 12 over the needle tip is accordingly achieved by the protective device 10.

Fig. 9, 13, 13a, 14, 14a, 15 and 15a depict another exemplary embodiment where the longitudinal finger receiving plate 29 includes on its inner surface one or more guiding tabs 50. In the illustrated example, the longitudinal finger receiving plate 29 includes three guiding tabs 50 which are axially offset. The guiding tabs 50 are configured and sized to engage and slide in two guiding grooves 51 provided on the outer surface of the tubular body 12. The two guiding grooves 51 are axially oriented and as they receive the guiding tabs 50 when the tubular body 12 moves towards the annular head 14, the finger receiving arrangement 27 is axially guided relative to the tubular body 12. This reduces radial play between the tubular body 12 and the annular head 14.

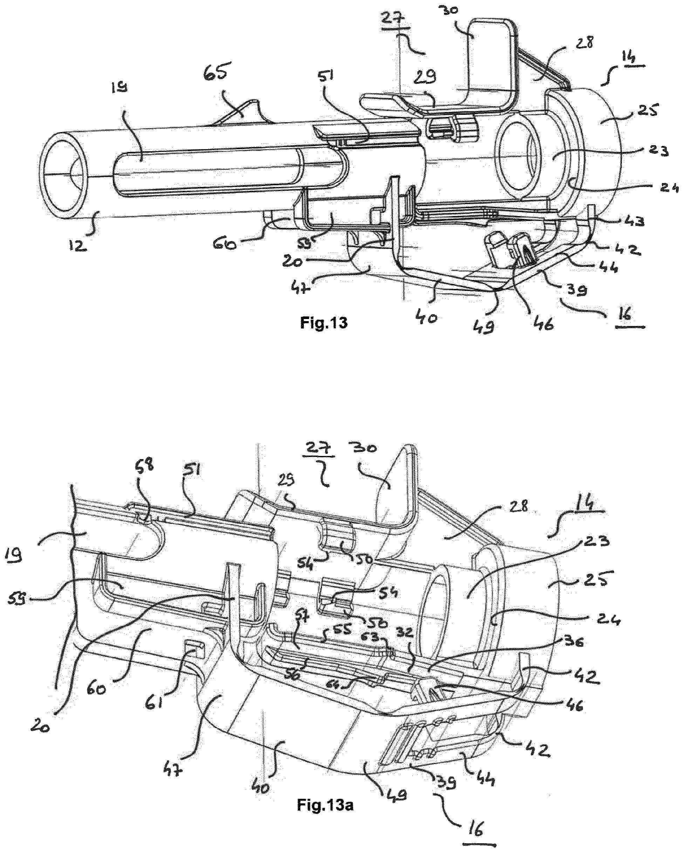

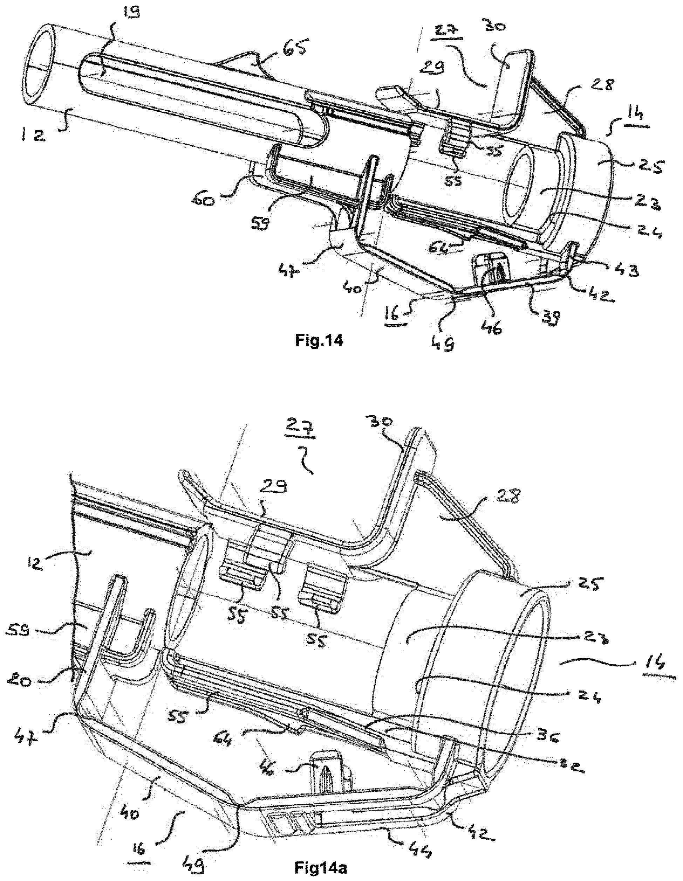

In some embodiments shown in Figs 13, 13a, 14, 14a, one of the guiding tabs 50 can include a teeth 54. As shown in Figs 13, 13a, 14, 14a, one guiding tab 50 on each side of finger receiving element 27 has a ramped teeth 54 at its free end. Each ramped teeth 54 is configured and sized to enter into a recess 58 formed in each of the guiding groove 51. In the illustrated embodiment, the tubular body 12 can also include a stop 65 whereon the longitudinal finger receiving plate 29 abuts when the tooth 54 click in the recesses 58. In some embodiments, the engagement of teeth 54 into the recesses 58 produces an audible and/or tactile signal indicating that locking is positively achieved.

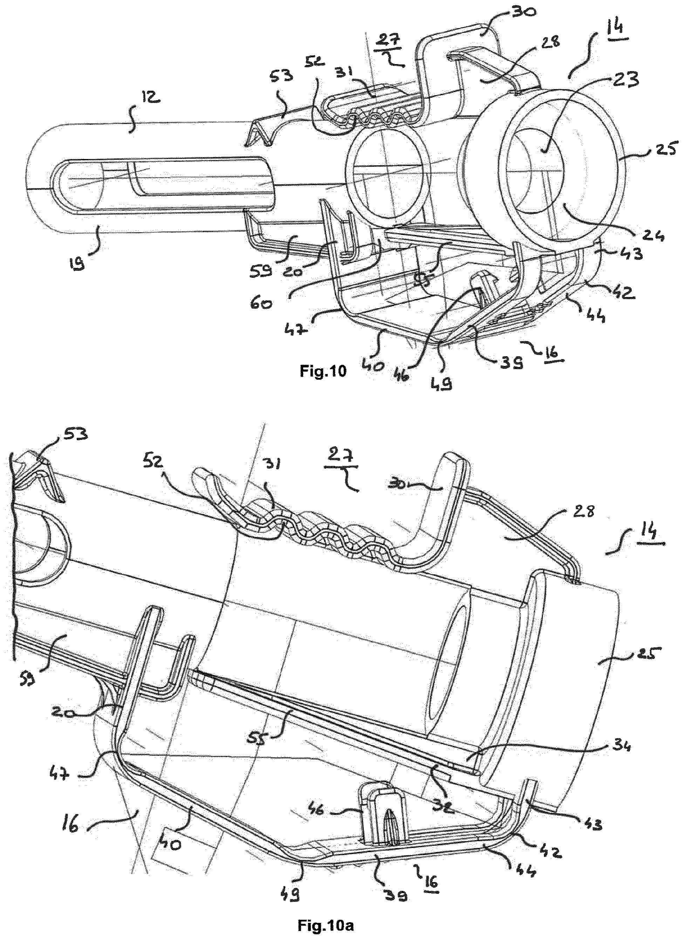

Fig. 10, 10a, 11, 11a, 12, 12a depict another exemplary embodiment of the invention. In this embodiment, the longitudinal finger receiving plate 29 includes one or more of transverse corrugations 52. The corrugations 52 are configured and sized to enter into resilient snap fit engagement with a rib 53 which protrudes from the tubular body 12.

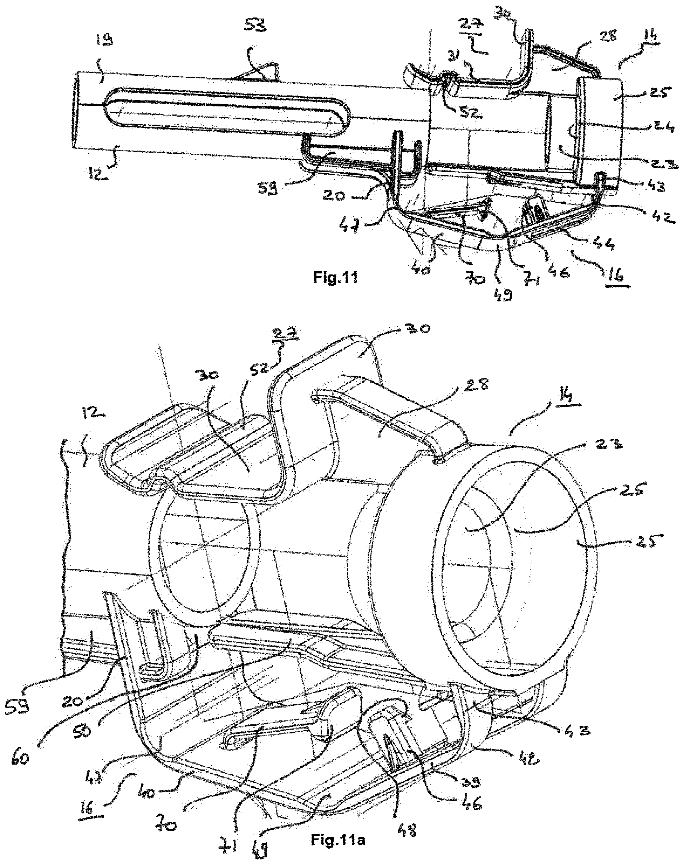

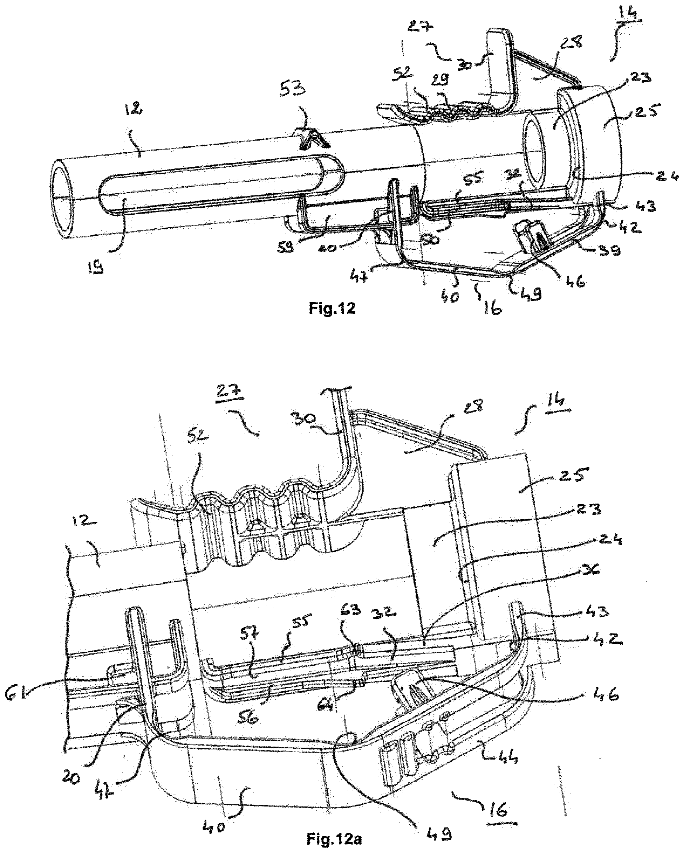

In some embodiments, the longitudinal finger plate 29 comprises a series of transverse corrugations 52 (see e.g. Figs 10, 10a, 12, 12a) while in some other embodiments the longitudinal finger plate 29 comprises a single transverse corrugations 52 (see e.g. Figs 11, 11a).

In some embodiments shown in Figs 10, 10a, 11, 11a, 12, 12a, 13, 13a, 14, the annular head 12 includes a longitudinal tongue 55 which extends distally from the locking member 32. The longitudinal tongue 55 has a substantially T shaped cross section with an axial rib 56and two wings 57 on each side of the axial rib 56. The tubular body 12 is further provided with two parallel guiding walls 59 which define a channel 60 configured and sized to receive the tongue 55. In the illustrated embodiment, the tab 20 includes a window 61 to allow the tongue 55 insertion between the two guiding walls 59.

While not shown, the tubular body 12 may be held in locked state via one of the corrugation 52 being engaged on the transverse rib 53 while the tongue 55 is engaged between the two guiding walls 59 thereby increasing the overall rigidity of the safety device 10 when in collapsed state. The elastic snap fit of the corrugation 52 on the rib 53 can provide an audible and/or tactile feedback to a user.

In one exemplary use, after an injection, a user pulls the free end of the longitudinal finger receiving plate 29 and by doing so the corrugation 52 is freed from the cross rib 53.

The safety device can be operated in the way illustrated on Fig. 8, that is to say that the user can then put a finger over the finger receiving element 27 and pushes on the actuation arm 16. The user can push on the intermediate hinge 49 so that both proximal link 39 and distal link 40 rotationally move towards the barrel 102. At the end of the push exerted by the user on the actuation arm 16, the locking hooks 46 engage onto the wings 36 thereby locking the tubular body 12 in an extended position where it shields the needle 101. It is noted that in the embodiment illustrated on Figs 11 and 11a, the operation of the safety device is independent from the drug delivery device. In other words, the piston flange abutting onto the annular ring does not have any effect on the safety device.

In some embodiments of the invention illustrated on Figs 12, 12a, 13, 13a, 14, 14a, the tongue 55 includes one or more teeth. As seen on Figs 12, 12a, 13, 13a the tongue 55 can include three teeth which are sized and configured to engage into three corresponding opening made in the tubular body 12. The tongue 55 can include two transverse teeth 63 and an outer dorsal tooth 64. In practice, each of the guiding wall 59 can include an opening 61 for each transverse tooth 63 and the tooth 64 can engage onto the tab 20. The teeth 63 and 64 thus offer a supplemental retentive force when the safety device is its locked state. In some embodiments (see e.g. Figs 14 and 14a), the tongue 55 includes an outer dorsal teeth 64. In some embodiments not shown, the tongue 55 includes one or two transverse teeth 63.

In some embodiments shown on Figs. 11 and 11a, the distal link 40 can include an arm 70 which extends at an angle from the inner surface of said distal link 40. The arm 70 includes at its end a substantially square shaped tab 71. The arm 70 and its tab 71 are configured and sized to enter into resilient snap fit engagement with the locking hooks 46 supported by the adjacent proximal link 39. When the safety device 10 is into its locked position that is to say a position where the proximal link 39 and the distal link 40 are brought closer to each other relative to the intermediate hinge 49 (as opposed to in line of each other when the safety device 10 is in its extended position), the arm 70 provides a supplemental retaining force. The retaining force in the locked position is further achieved by the corrugation 52 configured and sized to engage onto the rib 53 provided on the annular body 12.

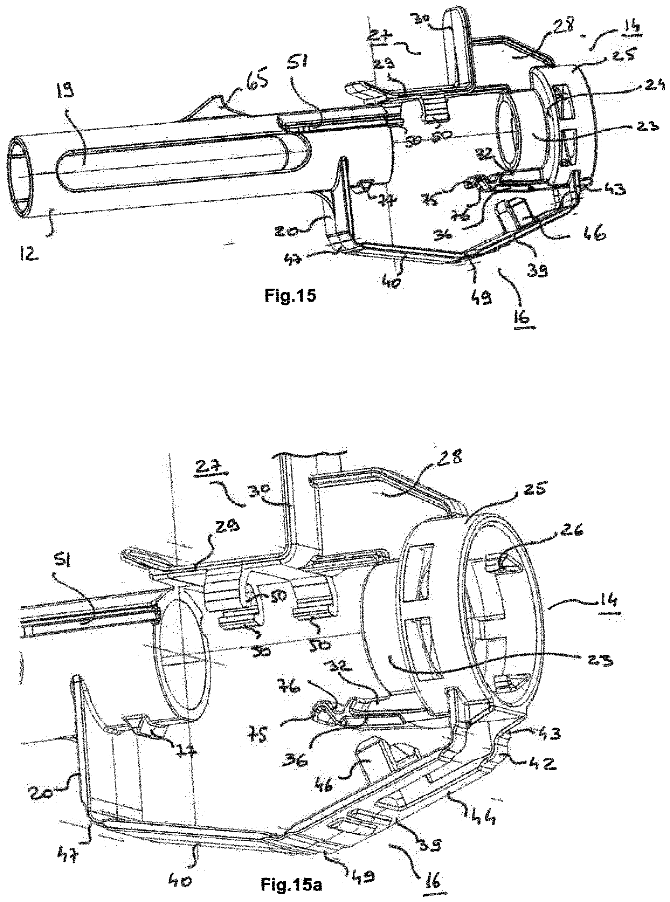

In some embodiments shown on Figs 15 and 15a, the annular head 14 includes a tab 75 which extends distally from the locking member 32. The tab 75 includes a transverse corrugation 76 which is sized and configured to engage on a rib 77 provided on the annular body 12. The corrugation 76 being engaged on the rib 77 provides an axially locking force when the actuation arm 16 is in its collapsed state.

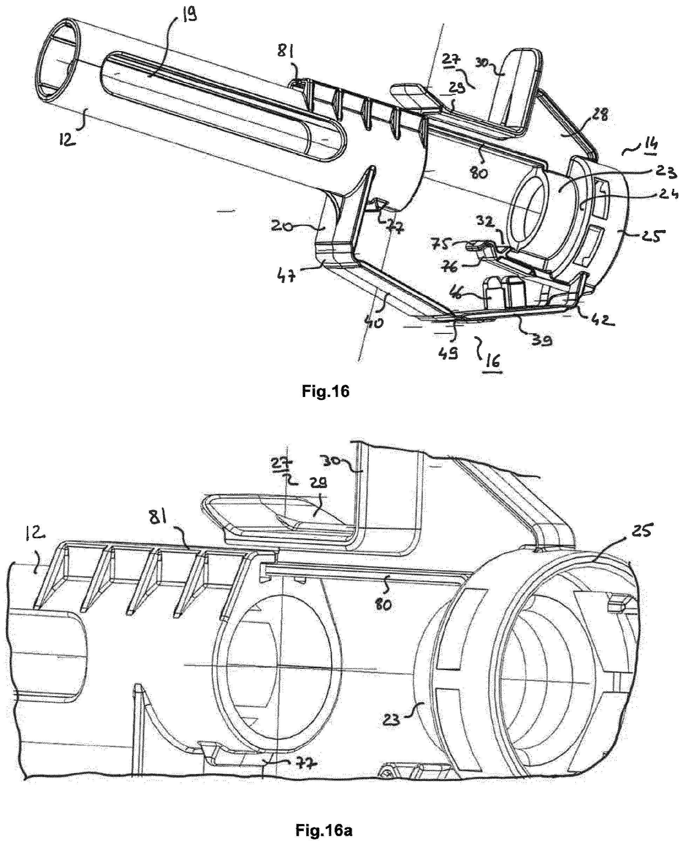

In some embodiments shown on Figs 16 and 16a, a T-shaped rib 80 formed along the inner face of the finger resting arrangement 27 mates with a complementary T-shaped groove 81 longitudinally formed along the tubular body 12. The T-shaped rib 80 originates at the end of the distal ring 23 and runs along the inner face of the finger resting arrangement 27 in a longitudinal direction. The T-shaped rib 81 runs on the tubular body 12 and may be flush with the distal end of said tubular body 12. A dove tail shape for the rib 80 and for the groove 81 can also be contemplated.

When the safety device 10 of Figs 16 and 16a is mounted on a drug delivery device and the safety device 10 is in its collapsed position (not shown), the T-shaped rib 80 is fully engaged into the T-shaped groove 80 thereby providing rigidity to the safety device 10.

When the safety device 10 moves from its collapsed position to its extended position, the T-shaped rib 80 slides in the T-shaped groove 81.

One may note that the safety device 10 of the embodiments depicted on Figs 10 to 16 relies on an action from the user to move the safety device 10 to its extended position irrespective of the position the plunger rod 110 and the position of plunger flange 114. In other words, the safety device 10 can be fitted on a drug delivery device where the quantity of drug delivered may vary according to parameters such as the patient weight, age or gender. Once the prescribed drug quantity, which may be less than the entire quantity of drug stored in the drug delivery device, is delivered, the user can move the safety device 10 in its extended position where the needle is shielded. One may further note that drug delivery device fitted with a safety device 10 as depicted on Figs 10 to 16 may include a plunger flange devoid of ramped surface.

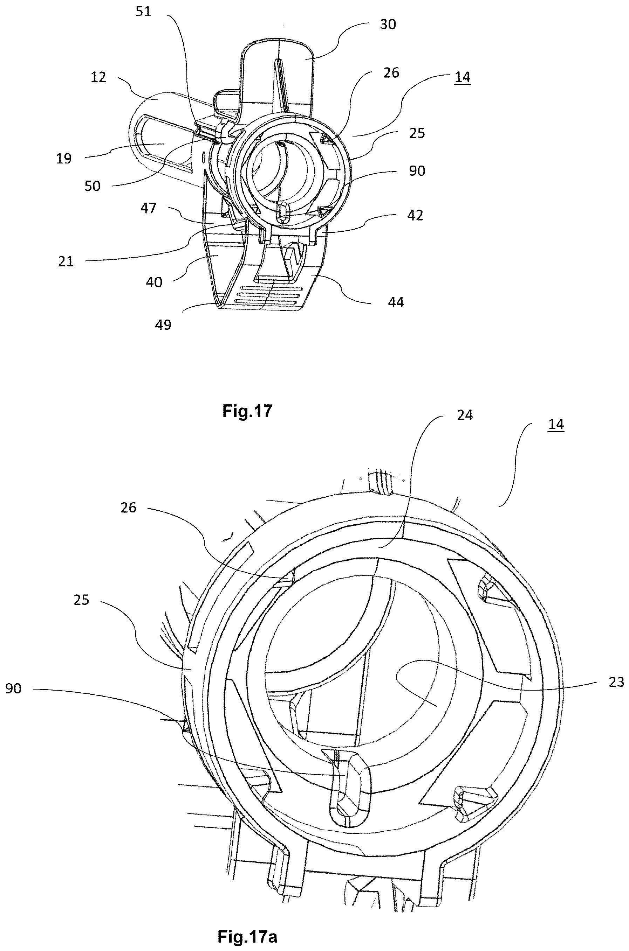

In some embodiments shown on Figs 17 and 17a, the safety device 10 can include one or more recesses configured to reduce the gross weight of the safety device 10. Reducing the gross weight further means that less raw plastic injection material is needed. Manufacturing cycles can be reduced as less material is required and cooling time is improved. In the illustrated embodiment of Figs 17 and 17a, a recess 90 is provided in the annular ring 14. The recess 90 can be positioned at the junction between the shoulder 24 and the distal ring 23. In one or more embodiments, the recess 90 can be located above the projecting locking member 32.

The terminology used herein is for the purpose of describing particular aspects only and is not intended to be limiting of the disclosure. As used herein, the singular forms "a," "an," and "the" are intended to include the plural forms as well, unless the context clearly indicates otherwise. As used herein, the term "and/or" includes any and all combinations of one or more of the associated listed items. It will be further understood that the terms "comprises," "comprising," "includes," and/or "including" when used herein specify the presence of stated features, integers, steps, operations, elements, and/or components, but do not preclude the presence or addition of one or more other features, integers, steps, operations, elements, components, and/or groups thereof.

It will be understood that, although the terms first, second, etc., may be used herein to describe various elements, these elements should not be limited by these terms. These terms are only used to distinguish one element from another. For example, a first element could be termed a second element, and, similarly, a second element could be termed a first element without departing from the scope of the present disclosure.

Relative terms such as "below" or "above" or "upper" or "lower" or "horizontal" or "vertical" may be used herein to describe a relationship of one element to another element as illustrated in the Figures. It will be understood that these terms and those discussed above are intended to encompass different orientations of the device in addition to the orientation depicted in the Figures. It will be understood that when an element is referred to as being "connected" or "coupled" to another element, it can be directly connected or coupled to the other element, or intervening elements may be present. In contrast, when an element is referred to as being "directly connected" or "directly coupled" to another element, there are no intervening elements present.

Unless otherwise defined, all terms (including technical and scientific terms) used herein have the same meaning as commonly understood by one of ordinary skill in the art to which this disclosure belongs. It will be further understood that terms used herein should be interpreted as having a meaning consistent with their meaning in the context of this specification and the relevant art and will not be interpreted in an idealized or overly formal sense unless expressly so defined herein.

It is to be understood that the present disclosure is not limited to the aspects described above and illustrated in the drawings; rather, the skilled person will recognize that changes and modifications may be made within the scope of the appended claims.

## Claims

1. A safety device (10) for mounting onto a drug delivery device (100) having a barrel (102) including with a flange (107) at its proximal end and an injection needle (101) at its distal end, and a piston rod (110) including a piston flange (114), the safety device (10) including:
a tubular body (12) extending along a longitudinal axis (A), the tubular body (12) being configured to receive the barrel (102),
an annular head (14) configured to abut against the flange (107),
one actuation arm (16) connecting the tubular body (12) and the annular head (14), said actuation arm having a proximal link (39) connected to the annular head (14) by a proximal hinge (42) and a distal link (40) connected to the tubular body (12) by a distal hinge (47), the proximal link (39) and the distal link (40) being connected by an intermediate hinge (49), the actuation arm (16) being movable between a collapsed position in which the tubular body (12) is maintained in a position proximally close to the flange uncovering the needle (101) and an extended position in which the tubular body (12) is maintained in a position distally distant from the annular head (14) shielding the needle (101),
one finger resting arrangement (27) connected to the annular head (14),
a locking means for maintaining the actuation arm (16) in a collapsed position and a locking means for maintaining the actuation arm (16) in an extended position.

2. The safety device (10) of claim 1, wherein the proximal link (39) includes at least one locking hooks (46) configured and sized to enter into resilient snap fit engagement with at least one wings (36) protruding from the annular head (14) to maintain the actuation arm (16) in the extended position.

3. The safety device (10) of claim 1 or claim 2, wherein the annular head (14) comprises a locking member (32) which includes a radially oriented wall (34) and two wings (36) extending laterally from the wall (34).

4. The safety device (10) of any of claims 1 to 3, wherein the annular head (14) includes a longitudinal tongue (55) which extends distally from the locking member (32) and is configured and sized to engage into a channel (60) defined by two guiding walls (59) provided on the tubular body (12).

5. The safety device of claim 4, wherein the longitudinal tongue (55) includes at least one tooth (63, 64) configured and sized to engage into on opening provide on the tubular body (12).

6. The safety device (10) of any of claims 1 to 5, wherein the annular head (14) includes a tab (75) which extends distally from the locking member (32), the tab (75) including a transverse corrugation (76) configured to engage and sized on a rib (77) provided on the annular body (12).

7. The safety device (10) of any of claims 1 or 6, wherein the intermediate hinge (49) inwardly overtakes the plan defined by the proximal hinge (42) and distal hinge (47) when the actuation arm (16) is in its extended position.

8. The safety device (10) of any of claims 1 to 7 wherein the annular head (14) includes a proximal ring (25) and a distal ring (23) jointed by a shoulder (24) whereon the flange (107) abuts.

9. The safety device (10) of any of claims 1 to 8, wherein at least one the hinge (42, 47, 49) is a living hinge.

10. The safety device (10) of any of claims 1 to 9, wherein the annular head comprises at least one locking tab (26) configured to retain the flange (107).

11. The safety device (10) of any of claims 1 to 10, wherein the tubular body (12) comprises a leg (21) extending in cantilever and having a catch (22) at its free end, the leg (21) being configured to deflect radially outward to allow the catch (22) to slide over the annular head (12) during mounting and to deflect radially inward after mounting completion so that the catch (22) axially retains the tubular body (12) relative to annular head (14).

12. The safety device (10) of any of claims 1 to 12, wherein the finger receiving member (27) comprises a longitudinal finger receiving plate (29) and a radial finger receiving plate (30).

13. The safety device (10) of claim 12, wherein the longitudinal finger receiving plate (29) comprises at least one corrugation (52) configured to engage onto a rib (53) protruding from the tubular body when the actuation arm is in its collapsed state.

14. The safety device (10) of any of claims 12 to 13, wherein the longitudinal finger receiving plate (29) comprises at least one guiding tab (50) extending from its inner face and configured to slidably engage with at least one guiding groove (51) provided on the tubular body (12).

15. The safety device (10) of claims 14, wherein at least one guiding tab (50) includes a teeth (54) positioned at its free end, the teeth (54) being configured and sized to snap in a recess (58) provided in the guiding groove (51).

16. The safety device (10) of any of claims 1 to 15, wherein the annular head (14) comprise a tongue (55) which extends distally in cantilever, configured to engage in a channel defined between two parallel guiding wall (59) extending outwardly from the tubular body (12).

17. The safety device (10) of claim 16, wherein the tongue (55) includes at least one teeth (63) configured to engage into a corresponding window made in the tubular body (12).

18. The safety device (10) one of any of claims 1 to 17, wherein the distal link (40) comprises an arm (70) extending from the distal link inner face, having at its free end, a tab (71) configured to enter into resilient snap fit engagement with the locking hooks (46).

19. The safety device (10) one of any of claims 1 to 18, wherein the safety device comprises a T-shaped rib (80) formed along the inner face of the finger resting arrangement (27) configured and sized to slide in a complementary T-shaped groove (81) longitudinally formed along the tubular body (12).

20. The safety device (10) one of any of claims 1 to 19, wherein the safety device (10) is formed in a single shot injection molding process.

21. The safety device (10) of any of claims 1 to 20, wherein, the safety device (10) includes at least one recess (90) positioned at the junction between the shoulder (24) and the distal ring (23).

22. A drug delivery device (100) having a barrel (102) including with a flange (107) at its proximal end and an injection needle (101) at its distal end, and a piston rod (110) including a piston flange (114) fitted with a safety device (100) of any one of claims 1 to 21, wherein the piston flange (114) includes a ramped surface (118) distally convergent configured to outwardly deflect the locking means maintaining the actuation arms (16,216) in a collapsed position.
